# EUROPEAN PATENT APPLICATION

(11) **EP 4 760 733 A1**
(43) Date of publication of application: **17.06.2026**
(21) Application number: 25221443.2
(22) Date of filing: 08.12.2025
(51) Int. Cl.: G16H 10/40, G16H 40/60

(54) **SYSTEM AND METHODS FOR ESTABLISHING AND MANAGING A PLATFORM LINK FOR LABORATORY EQUIPMENT**

(30) Priority: 12.12.2024 US 202463733306 P
(71) Applicant: Thermo Fisher Scientific India Private Limited, Powai, Mumbai, IN 400072 (IN); Thermo Electron Scientific Instruments LLC, Madison, WI 53711 (US)
(72) Inventor: BHAT, Pramod Ramachandra, Hyderabad (IN); RAINA, Rajesh, Fremont (US); PIRATLA, Anant, Hyderabad (IN); NESARGI, Sumeet, Hyderabad (IN)
(74) Representative: Boult Wade Tennant LLP

(57) **Abstract**

Systems and methods for managing a platform link. One example provides a scientific instrument support system includes a server storing a trained language model and an instrument computing device having a display device configured to provide a user interface, a memory including a platform link application providing an interface between the instrument computing device and a platform of network-based services, a transceiver, and an electronic processor communicatively connected to the display device, the memory, and the transceiver. The electronic processor is configured to receive a request associated with the platform link application, transmit, with the transceiver, the request to the trained language model, receive, with the transceiver and from the trained language model, a recommended command associated with the request, confirm an access level of a user submitting the request, and in response to confirming the access level of the user, execute the recommended command.

## Description

### Background

Scientific instruments may include a complex arrangement of movable components, sensors, input and output ports, energy sources, and consumable components. Lab systems may include many instruments and many individuals interacting with different instruments.

### Brief Description of the Drawings

Examples will be readily understood by the following detailed description in conjunction with the accompanying drawings. To facilitate this description, like reference numerals designate like structural elements. Aspects and instances are illustrated by way of example, not by way of limitation, in the figures of the accompanying drawings.
FIG. 1 is a block diagram of an example scientific instrument support system, in accordance with various examples.
FIG. 2 is a block diagram of an IPC and a server included in the scientific instrument support system of FIG. 1, in accordance with various examples.
FIG. 3 is a block diagram of a graphical user interface, in accordance with various examples.
FIG. 4 is a block diagram of a scientific instrument support module for performing support operations, in accordance with various examples.
FIG. 5 is a block diagram of a method of performing support operations, in accordance with various examples.
FIG. 6 is an example workflow for implementing a language model to process prompts, in accordance with various examples.
FIG. 7 is a block diagram of another scientific instrument support module for performing support operations, in accordance with various examples.
FIG. 8 is a block diagram of another method of performing support operations, in accordance with various examples.
FIG. 9 is a block diagram of another scientific instrument support module for performing support operations, in accordance with various examples.
FIG. 10 is a block diagram of another method of performing support operations, in accordance with various examples.
FIG. 11 is an example workflow for implementing a language model to process commands, in accordance with various examples.
FIG. 12 is an example workflow for implementing a language model to process queries, in accordance with various examples.

### Detailed Description

Disclosed herein are scientific instrument support systems, as well as related methods, computing devices, and computer-readable media. One example provides a scientific instrument support system includes a server storing a trained language model and an instrument computing device having a display device configured to provide a user interface, a memory including a platform link application providing an interface between the instrument computing device and a platform of network-based services, a transceiver, and an electronic processor communicatively connected to the display device, the memory, and the transceiver. The electronic processor is configured to receive a request associated with the platform link application, transmit, with the transceiver, the request to the trained language model, receive, with the transceiver and from the trained language model, a recommended command associated with the request, confirm an access level of a user submitting the request, and in response to confirming the access level of the user, execute the recommended command.

Another example provides a non-transitory computer readable medium storing instructions that, when executed by one or more electronic processing devices, perform a set of functions, the set of functions including receiving a request associated with a platform link application, where the platform link application provides an interface between an instrument computing device and a platform of network-based services, transmitting, with a transceiver, the request to a trained language model, receiving, with a transceiver and from the trained language model, a recommended command associated with the request, confirming an access level of a user submitting the request, and in response to confirming the access level of the user, executing the recommended command.

Another example provides a memory including a platform link application providing an interface between an instrument computing device and a platform of network-based services, and an electronic processor communicatively connected to the memory, the electronic processor configured to receive a request associated with the platform link application, provide the request to a trained language model, receive, from the trained language model, a recommended command associated with the request, confirm an access level of a user submitting the request, and in response to confirming the access level of the user, execute the recommended command.

The scientific instrument support examples disclosed herein may achieve improved lab management relative to conventional approaches. For example, as scientific laboratories increase in size, lab managers, scientists, and technicians experience difficulty viewing, monitoring, and managing instruments in a lab. Additionally, instruments and devices used for experiments may be located in different laboratories, adding communication latency to experiment time and status updates. Because each lab may include many individuals working in the lab, lab managers may experience issues determining availability of resources (such as, for example, instruments), and communicating instrument reservation status. Additionally, the growing complexity of labs causes issues for laboratory managers in identifying which devices require maintenance, remotely troubleshooting instruments, and communicating the nonoperational status of those instruments. Examples disclosed herein address these and other issues and, therefore, provide improvements to scientific instrument technology (e.g., improvements in the computer technology supporting such scientific instruments, among other improvements).

For example, instances and aspects disclosed herein provide, among other things, a system for querying the status and capabilities (e.g., operational status and data acquisition status) of scientific instruments and devices, troubleshooting how to correct errors related to scientific instruments and devices, controlling usage of scientific instruments and devices, executing commands for controlling scientific instruments and devices, or a combination thereof. Additionally, among other things, various ones of the examples disclosed herein may provide improvements to graphical user interface (GUI) technology. For example, GUIs provided herein may provide a status of some or all components (e.g., devices and scientific instruments) within a support system, as well as the availability of those components and potential troubleshooting and maintenance related to those components and the support system, which increases the efficiency of reserving and implementing projects on one or more components and associated computing and instrument usage, as well as efficiency of correcting errors related to the components. GUIs provided herein may also provide a plurality of options related to controlling components within a support system, enhancing user experience.

Various ones of the examples disclosed herein may improve upon conventional approaches to achieve the technical advantages of improved lab management and, consequently, improved operation of scientific instruments. Such technical advantages are not achievable by routine and conventional approaches, and all users of systems including such examples may benefit from these advantages (e.g., by assisting the user in the performance of a technical task, such as monitoring instrument data acquisition and operational status, by means of a guided human-machine interaction process). The technical features of the examples disclosed herein are thus decidedly unconventional in the field of lab management, as are the combinations of the features of the examples disclosed herein. The computational and user interface features disclosed herein do not only involve the collection and comparison of information but apply new analytical and technical techniques to change the management of instrument support systems. The present disclosure thus introduces functionality that neither a conventional computing device, nor a human, could perform.

Accordingly, the examples of the present disclosure may serve any of a number of technical purposes, such as managing a specific technical system or process; determining machine or instrument availability or status; optimizing load distribution in a computer network (e.g., by distributing projects and associated data processing across instruments included in one or more labs); providing faster processing of sensor data (e.g., by identifying available machine or instruments and associated data processing); troubleshooting and correcting errors associated with machines or instruments in a computer network; remotely controlling machines and instruments; identifying commands related to user queries; or a combination thereof.

The examples disclosed herein thus provide improvements to lab and instrument management technology (e.g., improvements in the computer technology supporting scientific instruments, among other improvements).

In the following detailed description, reference is made to the accompanying drawings that form a part hereof wherein like numerals designate like parts throughout, and in which is shown, by way of illustration, examples that may be practiced. It is to be understood that other examples may be utilized, and structural or logical changes may be made, without departing from the scope of the present disclosure. Therefore, the following detailed description is not to be taken in a limiting sense.

Various operations may be described as multiple discrete actions or operations in turn, in a manner that is most helpful in understanding the subject matter disclosed herein. However, the order of description should not be construed as to imply that these operations are necessarily order dependent. In particular, these operations may not be performed in the order of presentation. Operations described may be performed in a different order from the described example. Various additional operations may be performed, and/or described operations may be omitted in additional examples.

For the purposes of the present disclosure, the phrases "A and/or B" and "A or B" mean (A), (B), or (A and B). For the purposes of the present disclosure, the phrases "A, B, and/or C" and "A, B, or C" mean (A), (B), (C), (A and B), (A and C), (B and C), or (A, B, and C). Although some elements may be referred to in the singular (e.g., "a processing device"), any appropriate elements may be represented by multiple instances of that element, and vice versa. For example, a set of operations described as performed by a processing device may be implemented with different ones of the operations performed by different processing devices.

The description uses the phrases "an example," "various examples," and "some examples," each of which may refer to one or more of the same or different examples. Furthermore, the terms "comprising," "including," "having," and the like, as used with respect to examples of the present disclosure, are synonymous. When used to describe a range of dimensions, the phrase "between X and Y" represents a range that includes X and Y. As used herein, an "apparatus" may refer to any individual device, collection of devices, part of a device, or collections of parts of devices. The drawings are not necessarily to scale.

Unless the context of their usage unambiguously indicates otherwise, the articles "a," "an," and "the" should not be interpreted as meaning "one" or "only one." Rather these articles should be interpreted as meaning "at least one" or "one or more." Likewise, when the terms "the" or "said" are used to refer to a noun previously introduced by the indefinite article "a" or "an," "the" and "said" mean "at least one" or "one or more" unless the usage unambiguously indicates otherwise.

It should also be understood that although certain drawings illustrate hardware and software located within particular devices, these depictions are for illustrative purposes only. In some examples, the illustrated components may be combined or divided into separate software, firmware, and/or hardware. For example, instead of being located within and performed by a single electronic processor, logic and processing may be distributed among multiple electronic processors. Regardless of how they are combined or divided, hardware and software components may be located on the same computing device or may be distributed among different computing devices connected by one or more networks or other suitable communication links.

Thus, in the claims, if an apparatus or system is claimed, for example, as including an electronic processor or other element configured in a certain manner, for example, to make multiple determinations, the claim or claim element should be interpreted as meaning one or more electronic processors (or other element) where any one of the one or more electronic processors (or other element) is configured as claimed, for example, to make some or all of the multiple determinations. To reiterate, those electronic processors and processing may be distributed.

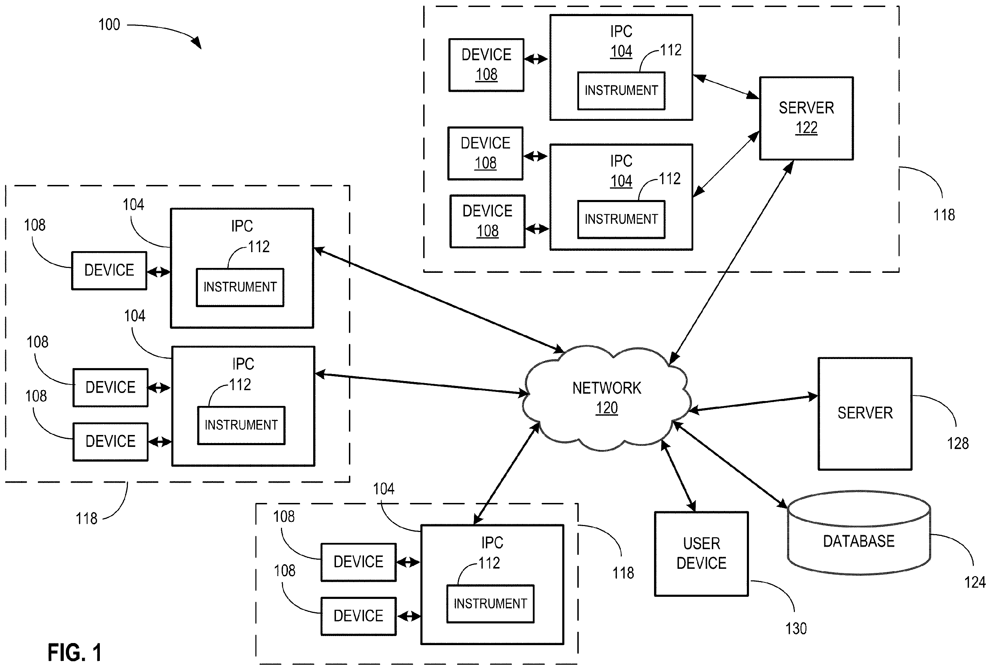

FIG. 1 is a block diagram of a scientific instrument support system 100 (henceforth referred to simply as support system 100) for performing support operations, in accordance with various examples. As illustrated in FIG. 1, the support system 100 includes a plurality of instrument personal computers (IPCs) 104 (also referred to herein as instrument computing devices) connected over a communication connection or network 120. One or more devices 108 are connected to each IPC 104. Devices 108 are physical entities that perform some function of sample preparation and/or sample analysis. For example, devices 108 may be physical devices having a serial number, a means of communicating with other external entities, and may include processors, memory, and firmware. Devices 108 may include, for example, sensors, detectors, actuators, spectrometers, spectrograms, oscilloscopes, electrometers, interferometers, and the like. The IPCs 104 may also include one or more instruments 112. Instruments 112 are logical containers that include a collection of devices 108. For example, devices 108 work together to perform operations and produce results. The logical collection of devices 108 (e.g., the instrument 112) prepares or analyzes inputs, such as blood samples or other biological samples, semiconductors, chemical compounds, solutions, food and drug samples, and the like. Each IPC 104 is connected to its respective device(s) 108 and may be configured to communicate uni-directionally or bi-directionally with the connected device(s) 108. Each IPC 104 may be connected to the device(s) 108 via wired or wireless communication mediums and/or protocols. Each IPC 104 may transmit commands to the connected device(s) 108, receive status signals from the device(s) 108, receive measurements from the device(s) 108, and the like, as described below in more detail. The IPCs 104, and their corresponding connected device(s) 108, may be organized into a plurality of scientific instrument groups 118. For example, a scientific instrument group 118 may include a liquid chromatography ("LC") instrument IPC 104 and a mass spectrometry ("MS") IPC 104, or a group 118 may include a LC device 108 and a MS device 108, which are connected to an IPC 104, wherein the combination constitutes or represents an instrument 112 as described herein. As illustrated in FIG. 1, a scientific instrument group 118 can include a standalone workstation IPC 104 or a group of IPCs 104 connected to an enterprise server 122. Each scientific instrument group 118 includes at least one IPC 104. The IPCs 104, the devices 108, and the instruments 112 may be more generally referred to as service components. The instruments 112 may include, for example, liquid chromatography instruments, gas chromatography instruments, ion chromatography instruments, mass spectrometry instruments, trace elemental instruments (e.g., inductively coupled plasma mass spectrometry, inductively coupled plasma optical emission spectroscopy, atomic absorption, etc.), capillary electrophoresis instruments, spectroscopy instruments, and the like. However, it should be understood that instruments 112 are not limited to those described herein, and other types of instruments are contemplated. For example, any type of instrument configured to follow or interact with the defined user interfaces may be supported and may be automatically detected (as described below) and managed within the support system 100. Furthermore, as also described in more detail below, in some examples, the support system 100 is configured to enable a user to manually add an instrument for management within the support system 100.

The support system 100 includes a database 124, a server 128, and a user device 130 communicatively coupled with the plurality of IPCs 104 (and with each other) through the communication network 120. In other examples, the plurality of IPCs 104, the server 128, and the database 124 communicate via one or more dedicated wire connections or other forms of wired or wireless electronic communication. It should be understood that the support system 100 may include fewer or additional components than those illustrated in FIG. 1. For example, the support system 100 may include fewer or more IPCs 104, multiple servers 128, multiple databases 124, or a combination thereof. In some instances, rather than including an independent database, the storage functionality of the database 124 is provided by the server 128. The components of the support system 100 may also communicate through one or more intermediary devices not illustrated in FIG. 1.

The server 128 may serve as a "control hub" for the plurality of IPCs 104 and, thus, establishes a computing platform and facilities communication with the IPCs 104, user devices 130, and other devices to provide one or more services. For example, the server 128 (i.e., one or more software applications executed on the server 128) communicates with each of the IPCs 104 by sending commands to the IPCs 104 to perform methods described herein over a wired connection, a wireless connection, or a combination thereof. Additionally, the server 128 may receive measurements and statuses of device(s) 108 and instrument(s) 112 from their respective IPCs 104 over a wired connection, wireless connection, or a combination thereof. The database 124 stores, for example, data indicating the status of components within the support system 100. For example, the database 124 may store a current status of the device(s) 108 and instrument(s) 112, historical statuses of the device(s) 108 and instrument(s) 112, measurements recorded by the device(s) 108 and instrument(s) 112, and the like. The server 128 (i.e., one or more software applications executed on the server 128) also provides data (e.g., through one or more GUIs) to the user devices 130 and, thus, acts as a central access point for obtaining information on the IPCs 104 and associated devices 108 and instruments 112 as well as controlling such IPCs 104, devices 108, and/or instruments 112 (in response to received user input via issuing commands or control signals to the IPCs 104, devices 108, and/or instruments 112). In other words, the server 128 may store and provide a collection of services (e.g., network-based services or web-based services) that may be hosted on a private or public cloud environment and used to manage and control laboratory equipment distributed in one or more locations (i.e., one or more labs).

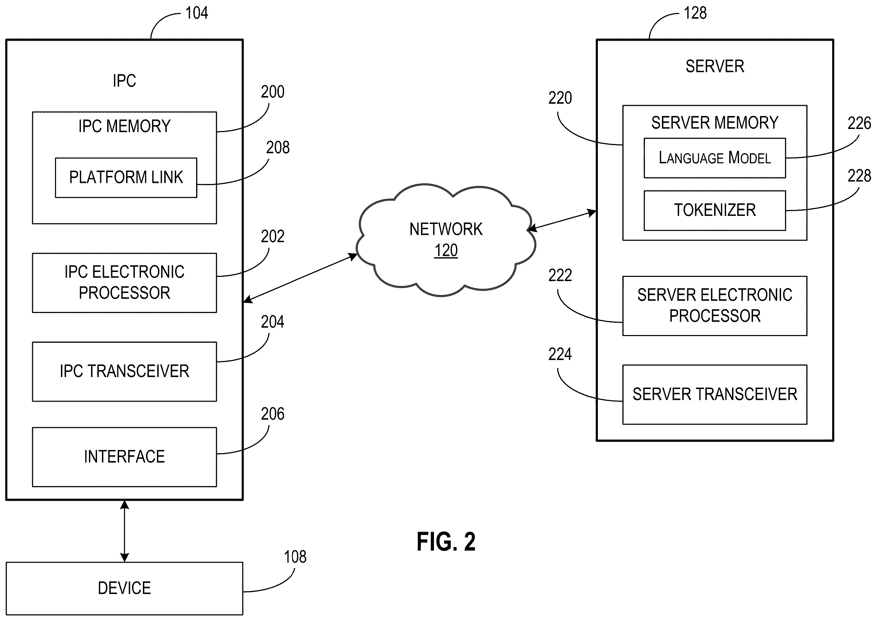

FIG. 2 is a block diagram of an example IPC 104 connected with the server 128 over the network 120 in more detail. The IPC 104 in FIG. 2 may represent any one of the IPCs 104 in FIG. 1. In the example of FIG. 2, the IPC 104 include an IPC memory 200, an IPC electronic processor 202, an IPC transceiver 204, and an interface 206.

The IPC memory 200 may include one or more memory devices such as random access memory (RAM) (e.g., static RAM (SRAM) devices, magnetic RAM (MRAM) devices, dynamic RAM (DRAM) devices, resistive RAM (RRAM) devices, or conductive-bridging RAM (CBRAM) devices), hard drive-based memory devices, solid-state memory devices, networked drives, cloud drives, or any combination of memory devices. In some examples, the IPC memory 200 may include non-transitory computer readable media having instructions thereon that, when executed by one or more processing devices (e.g., the IPC electronic processor 202), cause the IPC 104 to perform any appropriate ones of or portions of the methods disclosed herein.

As illustrated in FIG. 2, the IPC memory 200 may store a platform link application 208, which as described herein, acts as an interface or bridge between the IPC 104 and the server 128. For example, the platform link application 208 may manage the execution and control of services provided by the server 128 to the IPC 104 and may perform discovery and onboarding of the IPC 104 with the server 128. In some instances, the platform link application 208 also manages the execution, discovery, and onboarding of devices 108 and/or instruments 112 with the server 128 (using the IPC 104 as an intermediate communication device). In some examples, the platform link application 208 may provide a user interface (e.g., one or more GUIs provided through the interface 206 of the IPC 104) that allows a user to control and manage services provided via the server 128 to the IPC 104. For example, as described herein, the platform link application 208 may provide one or more user interfaces that allow a user (interfacing with the IPC 104 directly or remotely) to issue queries and requests regarding services provided via the platform (e.g., using a chat-bot based artificial intelligence (AI) tool), including controlling services provided via the platform, the platform link application 208, or a combination thereof. For example, the platform link application 208 may provide a set of services associated with the platform implemented via the server 128 (e.g., backend services), and users use the chat-bot tool to understand the ability and usability of these services provided via the platform link application 208. Thus, the interfaces respond to user queries to provide information, control services, and troubleshoot problems all while ensuring appropriate authentication as managed by the server 128 (i.e., the platform). These interfaces and associated functionality can be provided by the platform link application 208 or an associated but separate software tool installed on the IPC 104. As also described herein, the platform link application 208 may make application programming interface (API) calls to issue commands and provide data to or request data from other software applications executed by the IPC 104, the devices 108, and/or the instruments 112. For example, the IPC 104 may store and execute one or more driver applications that communicate and control the devices 108 and/or instruments 112 associated with the IPC 104 and the platform link application 208 may make API calls to such driver applications as part of responding to a query submitted via the chat-bot tool.

The IPC electronic processor 202 may include one or more digital signal processors (DSPs), application-specific integrated circuits (ASICs), central processing units (CPUs), graphics processing units (GPUs), cryptoprocessors (specialized processors that execute cryptographic algorithms within hardware), server processors, or any other suitable processing devices. The IPC electronic processor 202 may retrieve and implement instructions stored by the IPC memory 200 to perform any appropriate ones of or portions of the methods disclosed herein.

The IPC transceiver 204 may be any transmitter and receiver device that provides communication capabilities to the IPC 104. The IPC 104 may communicate over the network 120 using the IPC transceiver 204 (for example, with the server 128).

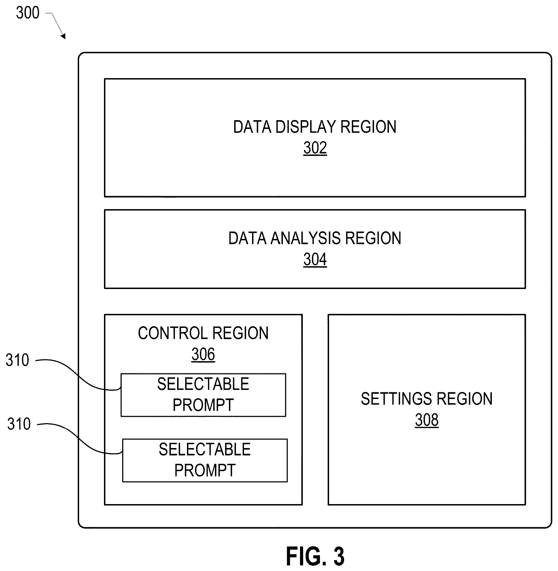

The interface 206 may include any visual indicators, such as a heads-up display, a computer monitor, a projector, a touchscreen display, a liquid crystal display (LCD), a light-emitting diode display, or a flat panel display. The interface 206 may also include a user interface for retrieving inputs, as described with respect to FIG. 3.

In the example of FIG. 2, the server 128 includes a server memory 220, a server electronic processor 222, and a server transceiver 224. The server memory 220 may include one or more memory devices such as random access memory (RAM) (e.g., static RAM (SRAM) devices, magnetic RAM (MRAM) devices, dynamic RAM (DRAM) devices, resistive RAM (RRAM) devices, or conductive-bridging RAM (CBRAM) devices), hard drive-based memory devices, solid-state memory devices, networked drives, cloud drives, or any combination of memory devices. In some examples, the server memory 220 may include non-transitory computer readable media having instructions thereon that, when executed by one or more processing devices (e.g., the server electronic processor 222), cause the server 128 to perform any appropriate ones of or portions of the methods disclosed herein.

Additionally, the server memory 220 may store a language model 226 and a tokenizer 228. The language model 226 is a machine learning (or machine-learned) model configured to perform speech recognition, machine translation, natural language generation, information retrieval, or the like. The language model 226 may be a small language model (SLM), a large language model (LLM), or the like. As one example, the language model 226 may be the Phi-2 language model. The language model 226 may work in conjunction with the tokenizer 228 that "tokenizes" or breaks down received text into lexical tokens for processing by the language model 226. In some examples, the language model 226 and/or the tokenizer 228 may be stored on separate servers and one or more of these components may be stored on one or more servers separate from the server 128 providing the services described herein. For example, the language model 226 and the tokenizer 228 may be stored on a web server that the IPC 104 is configured to communicate with (e.g., via the platform link application 208). Also, in some embodiments, the tokenizer 228 may be stored locally on the IPC 104 and configured to break-down text (e.g. queries submitted via the chat-bot tool) and submit the resulting tokens to the language model 226.

The server electronic processor 222 may include one or more digital signal processors (DSPs), application-specific integrated circuits (ASICs), central processing units (CPUs), graphics processing units (GPUs), cryptoprocessors (specialized processors that execute cryptographic algorithms within hardware), server processors, or any other suitable processing devices. The server electronic processor 222 may retrieve and implement instructions stored by the server memory 220 to perform any appropriate ones of or portions of the methods disclosed herein.

The server transceiver 224 may be any transmitter and receiver device that provides communication capabilities to the server 128. The server 128 may communicate over the network 120 using the server transceiver 224 (for example, with the IPC 104).

FIG. 3 depicts an example GUI 300 that may be used in the performance of some or all of the support methods disclosed herein, in accordance with various examples. The GUI 300 may be provided on a display device, such as the interface 206 of FIG. 2. A user may interact with the GUI 300 using any suitable input device (e.g., a mouse, a keyboard, a touch screen, etc.) and input technique (e.g., movement of a cursor, motion capture, facial recognition, gesture detection, voice recognition, actuation of buttons, etc.).

The GUI 300 may include a data display region 302, a data analysis region 304, a control region 306, and a settings region 308. The particular number and arrangement of regions depicted in FIG. 3 is simply illustrative, and any number and arrangement of regions, including any desired features, may be included in a GUI 300.

The data display region 302 may display data generated by a scientific instrument or device (e.g., a device 108). For example, the data display region 302 may display a status of the device 108 that is connected to the IPC 104 of FIG. 2.

The data analysis region 304 may display the results of data analysis (e.g., the results of analyzing the data illustrated in the data display region 302 and/or other data). In some examples, the data display region 302 and the data analysis region 304 may be combined in the GUI 300 (e.g., to include data output from a scientific instrument, and some analysis of the data, in a common graph or region).

The control region 306 may include options that allow the user to manage a scientific instrument (e.g., a device 108). For example, the control region 306 may include control options for operating the device 108. In some instances, the control region 306 may provide options selectable by a user for operating or altering the device 108. For example, the control region 306 may include one or more selectable prompts 310, as described below in more detail.

The settings region 308 may include options that allow the user to manage the features and functions of the GUI 300 (and/or other GUIs) and/or perform common computing operations with respect to the data display region 302 and data analysis region 304 (e.g., saving data on a storage device, such as the IPC memory 200 and/or the server memory 220, sending data to another user, labeling data, etc.).

As described herein, the language model 226 may be used to provide a chat-bot AI tool that responds to queries submitted via the platform link application 208. For example, in some implementations, the language model 226 is trained using documentation related to the IPC 104, the device(s) 108 and instrument(s) 112 associated with the IPC 104, the server 128, services provided via the server 128, or a combination thereof. The documentation may include manuals or other published documents relating to onboarding and registering an IPC to the server 128, troubleshooting issues associated an IPC 104, the server 128, or a connection therebetween, or the like. In some examples, the documentation may be created specifically for training the language model 226 and may include pairs of queries and corresponding answers. The language model 226 may be trained offline or online. For example, feedback (e.g., provided via a user directly or obtained indirectly based on a response to a query, such as operation of the system 100 performed in response to a query) may be used to provide continued training of the language model 226.

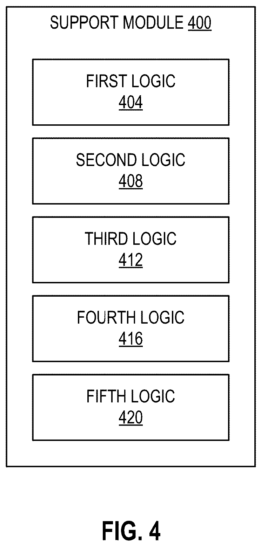

FIG. 4 is a block diagram of a scientific instrument support module 400 for performing support operations including training the language model 226, in accordance with various examples. The scientific instrument support module 400 may be, for example, the server 128. The scientific instrument support module 400 may be implemented by circuitry (e.g., including electrical and/or optical components), such as a programmed computing device. The logic of the scientific instrument support module 400 may be included in a single computing device or may be distributed across multiple computing devices that are in communication with each other as appropriate.

The scientific instrument support module 400 may include first logic 404, second logic 408, third logic 412, fourth logic 416, and fifth logic 420. As used herein, the term "logic" may include an apparatus that is to perform a set of operations associated with the logic. For example, any of the logic elements included in the support module 400 may be implemented by one or more computing devices programmed with instructions to cause one or more processing devices of the computing devices to perform the associated set of operations. In a particular example, a logic element may include one or more non-transitory computer-readable media having instructions thereon that, when executed by one or more processing devices of one or more computing devices, cause the one or more computing devices to perform the associated set of operations. As used herein, the term "module" may refer to a collection of one or more logic elements that, together, perform a function associated with the module. Different ones of the logic elements in a module may take the same form or may take different forms. For example, some logic in a module may be implemented by a programmed general-purpose processing device, while other logic in a module may be implemented by an application-specific integrated circuit (ASIC). In another example, different ones of the logic elements in a module may be associated with different sets of instructions executed by one or more processing devices. A module may not include all of the logic elements depicted in the associated drawing; for example, a module may include a subset of the logic elements depicted in the associated drawing when that module is to perform a subset of the operations discussed herein with reference to that module.

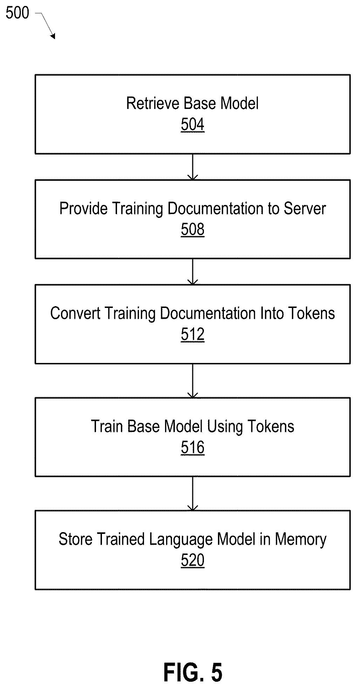

The logic elements illustrated in FIG. 4 may be configured to perform the method 500 illustrated in FIG. 5. Although the operations of the method 500 may be illustrated with reference to particular examples disclosed herein (e.g., the scientific instrument support system 100 discussed herein with references to FIGS. 1 and 2, the scientific instrument support modules 400 discussed herein with reference to FIG. 4, the GUI 300 described herein with reference to FIG. 3), the method 500 may be used in any suitable setting to perform any suitable support operations. Operations are illustrated once each and in a particular order in FIG. 5, but the operations may be reordered and/or repeated as desired and appropriate (e.g., different operations performed may be performed in parallel, as suitable).

As illustrated in FIG. 5, the first logic 404 may be configured to retrieve a base model (at 504). For example, the server electronic processor 222 may acquire a base model, such as the Phi-2 language model, from the database 124 or another source.

The second logic 408 may be configured to provide training documentation to the server 128 (at 508). For example, the server electronic processor 222 may be configured to acquire documentation related to the IPCs 104, the device(s) 108, the instrument(s) 112, the server 128, services provided by the server 128 (e.g., the platform provided by the server 128), or a combination thereof. The documentation may include, for example, user manuals, troubleshooting documentation, software specifications or APIs, other documentation that describes the operation and maintenance of associated IPCs 104, devices 108, server 128, and/or instruments 112, or any combination thereof. In some instances, the documentation may also or alternatively include pairs of questions/queries and answers that relate to the IPCs 104, the device(s) 108, the instrument(s) 112, the server 128 or service provided by the server 128, or a combination thereof. The pair may include text-based query and answer pairs, text-based queries paired with command or control signals, or a combination thereof. For example, the question "What services does the platform link application provide?" may be paired with the answer "The platform link application provides a set of web-based services for chromatography data systems, including backend services associated with laboratory equipment platform." Similarly, the question "Could you please restart the instrument diagnostic service?" may be paired with the appropriate command logic (e.g., in machine code or source code) for instructing the platform link application 208 (or a separate application stored on the IPC 104) to control the IPC 104 or a connected device 108 and/or instrument 112 to respond to the submitted query. Accordingly, the language model 226 can be trained to not only provide text-based answers but also directly control the IPC 104 to respond to a submitted question/query.

The third logic 412 may be configured to convert the training documentation into tokens (at 512). For example, the server electronic processor 222 may be configured to implement the tokenizer 228 to convert the text of the training documentation into lexicon tokens compatible as input to the base model.

The fourth logic 416 may be configured to train the base model using the tokens (i.e., fine-tune the base model to create a fine-tuned model as the language model 226) (at 516). For example, the server electronic processor 222 may be configured to provide the tokens generated using the training documents to the base model, thereby generating the language model 226. In some instances, additional fine-tuning parameters may also be applied to the base model when generating the language model 226. It should be understood that training may occur over multiple iterations.

The fifth logic 420 may be configured to store the trained language model in a memory (at 520, FIG. 5). For example, the server electronic processor 222 may be configured to store the language model 226 in the server memory 220. However, as noted above, in some implementations, the language model 226 may be stored in a separate device from the server 128, such as, for example, a web server.

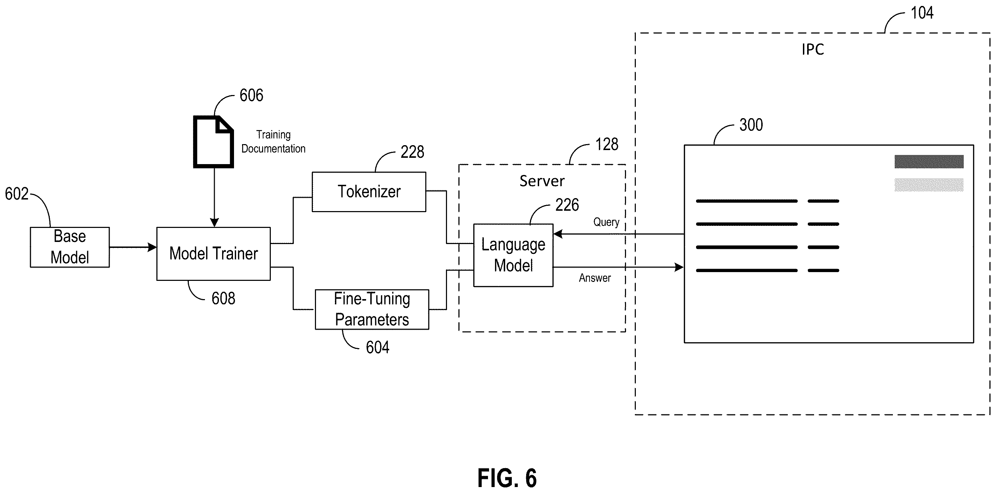

FIG. 6 illustrates additional details regarding the training of the language model 226 and, in particular, illustrates an example workflow for training the language model 226, which may be implemented via the logic elements of FIG. 4 in some implementations. As illustrated in FIG. 6 and as described with respect to FIGS. 4 and 5, a base model 602 is obtained (e.g., downloaded), such as, for example, a Phi 2 base model downloaded using a transformers library and initialized in a memory. Fine-tuning parameters 604 and an instance of the tokenizer 228 are also defined. The fine-tuning parameters 604 may include, for example, Parameter-Efficient Fine-Tuning (PEFT) parameters, such as learning rate, epochs, batch size, and the like. The fine-tuning parameters 604 may also include a scaling factor for low-rank matrices, indications of which layers of the language model 226 may be adapted, a dropout rate applied to low-rank adaptation, and the like. Training documentation 606 (also referred to as a training data set) is also loaded into memory along with a model trainer 608. As previously noted, the training documentation 606 may include a set of question and answer pairs related to the platform link application 208, which may be stored in a structured text file format, such as, for example, a comma-separated file or a character encoded format, such as, for example, a UTF-8 format. The base model 602, the instance of the tokenizer 228, and the fine-tuning parameters 604 are processed by the model trainer 608 to retrain (fine-tune) the base model 602. After training is complete (i.e., with multiple iterations), the language model 226 is obtained as a fine-tuned model. As illustrated in FIG. 6, the language model 226 may be stored on the server 128. To interact with the language model 226, a construct, often referred to as a pipeline, is provided and the language model 226 is wrapped in this construct. The wrapped model 226 can be hosted on the server 128 (or a separate web server), wherein other software applications, such as the platform link application 208 installed on the IPC 104, can interact with the language model 226, such as through one or more API calls. For example, a RESTful API may be used by the platform link application 208 to interface with the fine-tuned language model 226 over the network 120 and communicate queries obtained through one or more user graphical user interfaces (GUIs) 300 of the platform link application 208 to the model 226 and receive answers, which may be provided within the GUIs 300 and/or, when such answer include command instructions, executed via one or more software applications executed on the IPC 104.

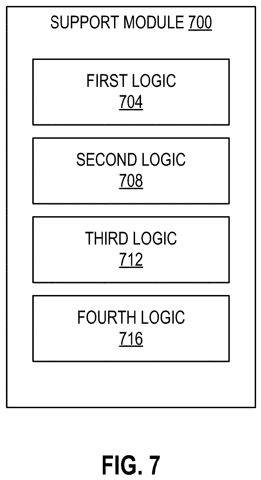

In some examples, the IPC electronic processor 202 and/or the server electronic processor 222 may authenticate a user of the support system 100 as part of using or interacting with the learned model 226. FIG. 7 is a block diagram of a scientific instrument support module 700 for performing support operations including authenticating a user of the support system 100 in accordance with various examples. The scientific instrument support module 700 may be, for example, the server 128. The scientific instrument support module 700 may be implemented by circuitry (e.g., including electrical and/or optical components), such as a programmed computing device. The logic of the scientific instrument support module 700 may be included in a single computing device or may be distributed across multiple computing devices that are in communication with each other as appropriate. It should be understood that the authentication described herein may be performed at one or more points during the support operations described herein. For example, authentication may be performed as part of execution or log-in of the platform link application 208, related to submission of a query to the learning mode 226, related execution of a response to a submitted query (including whether a response can be provided to a user and/or executed on behalf of the user), or a combination thereof.

The scientific instrument support module 700 may include first logic 704, second logic 708, third logic 712, and fourth logic 716. Any of the logic elements included in the support module 700 may be implemented by one or more computing devices programmed with instructions to cause one or more processing devices of the computing devices to perform the associated set of operations. In a particular example, a logic element may include one or more non-transitory computer-readable media having instructions thereon that, when executed by one or more processing devices of one or more computing devices, cause the one or more computing devices to perform the associated set of operations. Different ones of the logic elements in a module may take the same form or may take different forms. For example, some logic in a module may be implemented by a programmed general-purpose processing device, while other logic in a module may be implemented by an application-specific integrated circuit (ASIC). In another example, different ones of the logic elements in a module may be associated with different sets of instructions executed by one or more processing devices. A module may not include all of the logic elements depicted in the associated drawing; for example, a module may include a subset of the logic elements depicted in the associated drawing when that module is to perform a subset of the operations discussed herein with reference to that module.

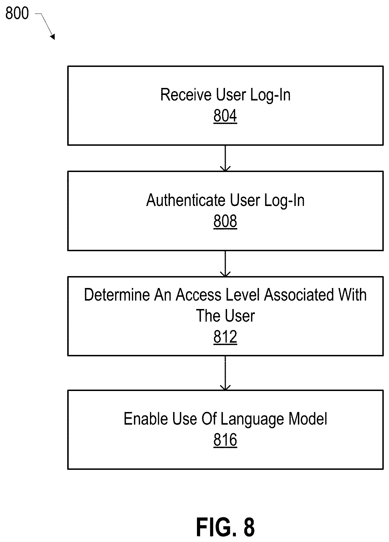

The logic elements of FIG. 7 may be configured to perform the method of FIG. 8. For example, FIG. 8 is a flow diagram of a method 800 of performing support operations, in accordance with various examples. Although the operations of the method 800 may be illustrated with reference to particular examples disclosed herein (e.g., the scientific instrument support system 100 discussed herein with references to FIGS. 1 and 2, the scientific instrument support modules 400 discussed herein with reference to FIG. 4, the GUI 300 described herein with reference to FIG. 3), the method 800 may be used in any suitable setting to perform any suitable support operations. Operations are illustrated once each and in a particular order in FIG. 8, but the operations may be reordered and/or repeated as desired and appropriate (e.g., different operations performed may be performed in parallel, as suitable

As illustrated in FIG. 8, the first logic 704 may be configured to receive data identifying a user, such as, for example, a user log-in. For example, a user of the IPC 104 may log-in to the scientific instrument support system 100 (e.g., the platform link application 208) using a user identifier (e.g., a username and password, an account number, etc.). The user may attempt to log in to the support system 100 by interacting with the GUI 300 using one or more input devices, wherein the GUI 300 may prompt the user to enter identifying data or obtain applicable data as part of single-sign-on functionality.

The second logic 708 may be configured to authenticate the identifying data (e.g., the user log-in). For example, a user identifier and authenticating data (e.g., a password) may be transmitted by the IPC 104 to the server 128. The server electronic processor 222 may be configured to compare the user identifier and/or authenticating data to user profiles stored on the server 128 (for example, by the 220) or by another storage device (for example, the database 124). In response to the user identifier and authenticating data matching a user profile, the user log-in is authenticated by the server electronic processor 222 and the software application or module submitting the authentication request to the server 128 may receive a confirmation of the same, and, in response to such a confirmation, the software application or module may perform one or more actions. In response to the user identifier or authenticating data not matching a user profile, the user log-in is not authenticated and the server electronic processor 222 may transmit a notification to the software application or module submitting the authentication request denying the log-in (e.g., to the IPC 104 or the platform link application 208) and/or a request for the user to register with the support system 100 or re-try the authentication process.

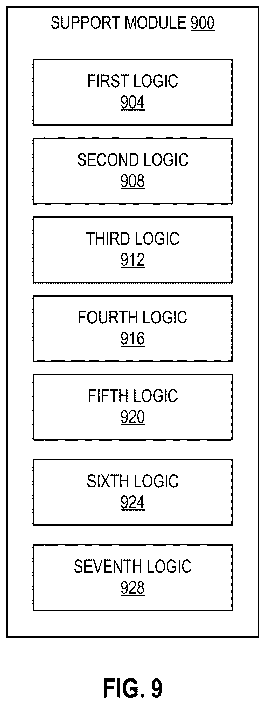

The third logic 712 may be configured to determine an access level (e.g., an authentication level, an interaction level) associated with the user. For example, the user profile that matches the user identifier may indicate the access level of the user. Answers to queries and/or recommended commands provided by the language model 226 (as described in more detail with respect to FIGS. 9-11) may be based on (e.g., limited by) the authentication or access level of the user. In some embodiments, access level information may be provided as part of the response from the server 128 regarding authentication of a user (as described above with respect to the second logic 708). Alternatively or in addition, a confirmation may be provided regarding user authentication only after determining an access level of the authenticated user and whether the access level satisfies a required level of a service being requested as part of the authentication request.

For example, the fourth logic 716 may be configured to enable use of the language model based on the access level. For example, commands recommended by the language model 226 (or performed by the IPC 104 in conjunction with the language model 226) may be limited for a user with a low access level, such as being limited to only checking a status of a device 108 or commanding the device 108 to perform an operation. Commands recommended by the language model 226 for a user with a high access level may be unrestricted or less restricted, such as permitting the altering of settings of the device 108. Recommended and performed commands output via the language model 226 may also be restricted based on a role of the user as indicated by the user identifier (which may dictate an access level of the user). As one example, only a maintenance worker may be permitted to troubleshoot the device 108 using the language model 226. In some instances, when the user of the IPC 104 fails to log-in (e.g., a username and password do not match), the server 128 (and therefore, the language model 226) does not respond to commands or queries. Also, it should be understood that a user may be initially authenticated to use the platform link application 208 and additional authentication may be determined and applied while the user interacts with the platform link application 208, such as, for example, confirming whether a recommend command or operation (as output via the language model 226) is performed or provided to the user in response to a particular query/request. In such an example, the recommended command provided by the language model 226 may only be provided to the user (including executed on behalf of the user) in response to proper authentication of the user (including verification of the user's access level and/or role).

After the language model 226 is trained, the language model 226 is implemented to respond to queries and commands from a user as provided via one or more user interfaces 300 of the platform link application 208 installed on the IPC 104. For example, FIG. 9 is a block diagram of a scientific instrument support module 900 for performing support operations including responding to user input received via one or more user interfaces of the platform link application 208, in accordance with various examples. The scientific instrument support module 900 may be, for example, implemented on an IPC 104. The scientific instrument support module 900 may be implemented by circuitry (e.g., including electrical and/or optical components), such as a programmed computing device. The logic of the scientific instrument support module 900 may be included in a single computing device or may be distributed across multiple computing devices that are in communication with each other as appropriate.

The scientific instrument support module 900 may include first logic 904, second logic 908, third logic 912, fourth logic 916, fifth logic 920, sixth logic 924, and seventh logic 928. Any of the logic elements included in the support module 700 may be implemented by one or more computing devices programmed with instructions to cause one or more processing devices of the computing devices to perform the associated set of operations. In a particular example, a logic element may include one or more non-transitory computer-readable media having instructions thereon that, when executed by one or more processing devices of one or more computing devices, cause the one or more computing devices to perform the associated set of operations. Different ones of the logic elements in a module may take the same form or may take different forms. For example, some logic in a module may be implemented by a programmed general-purpose processing device, while other logic in a module may be implemented by an application-specific integrated circuit (ASIC). In another example, different ones of the logic elements in a module may be associated with different sets of instructions executed by one or more processing devices. A module may not include all of the logic elements depicted in the associated drawing; for example, a module may include a subset of the logic elements depicted in the associated drawing when that module is to perform a subset of the operations discussed herein with reference to that module.

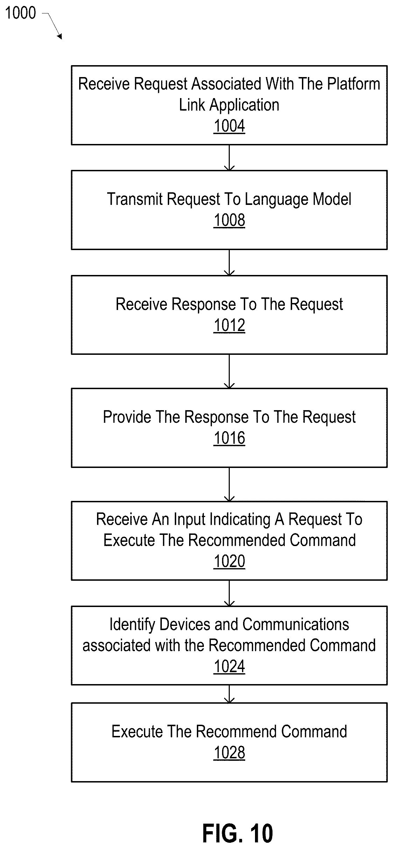

The logic elements of FIG. 9 may be configured to perform the method of FIG. 10. For example, FIG. 10 is a flow diagram of a method 1000 of performing support operations, in accordance with various examples. Although the operations of the method 1000 may be illustrated with reference to particular examples disclosed herein (e.g., the scientific instrument support system 100 discussed herein with references to FIGS. 1 and 2, the scientific instrument support modules 400 discussed herein with reference to FIG. 4, the scientific instrument support modules 700 discussed herein with reference to FIG. 7, the GUI 300 described herein with reference to FIG. 3), the method 1000 may be used in any suitable setting to perform any suitable support operations. Operations are illustrated once each and in a particular order in FIG. 10, but the operations may be reordered and/or repeated as desired and appropriate (e.g., different operations performed may be performed in parallel, as suitable).

As illustrated in FIG. 10, the first logic 904 may be configured to receive a request associated with the platform link application 208 (at 1004). For example, with reference to FIG. 2, the interface 206 may provide a chat box (e.g., the chat-bot tool) via the GUI 300. The IPC 104 may receive, via an input device of the interface 206, a request associated with the platform link application 208. The requests may be text typed by a user into, for example, the chat box. Example requests may include asking for services provided by the server 128 (via the platform link application 208), asking for information relating to registration with the platform link application 208, troubleshooting related to the platform link application 208, requests or commands for the platform link application 208, asking the status of the device 108, asking to reserve the device 108, asking for the capabilities of the device 108, a request or command to perform an operation using the device 108, troubleshooting related to the device 108 (e.g., asking why the device 108 is not functioning), or other questions related to the platform link application 208 and/or the device 108. In some instances, the request may be a request for a status report related to IPCs 104, devices 108, and/or instruments 112 registered with the support system 100 via the platform link application 208. A status report may be a report detailing diagnostic data related to service components registered with the platform link application 208, previous troubleshooting and/or maintenance of service components registered with the platform link application 208, a reservation status of service components registered with the platform link application 208, capabilities of service components registered with the platform link application 208, a usage history of service components registered with the platform link application 208, and the like. It should be understood that the request may be submitted via text or in other formats. For example, in some embodiments, a request may be submitted audibly where the audio input may be converted to text and then processed as described herein. For example, a microphone of the IPC 104 may be used to obtain verbal requests and responses may be provided via the GUI, a speaker, or a combination thereof.

The second logic 908 may be configured to transmit the request to the language model 226 (at 1008). For example, the IPC 104 may be configured to transmit the received request using the IPC transceiver 204 to the server 128. The server 128 inputs the request to the language model 226. In some instances, the request may first be processed into lexicon tokens by the tokenizer 228.

The third logic 912 may be configured to receive a response to the request (at 1012). For example, the language model 226 processes the request and identifies a response to the request. The server 128 transmits the response to the IPC 104 using the server transceiver 224. The IPC 104 receives, via the IPC transceiver 204, a response to the request from the server 128. The response may be, for example, a section of the training documentation that is related to or answers the request. The response may indicate a status of one or more service components. The response may relate to services provided by the server 128 (via the platform link application 208). The response may be a recommended operation or command to perform to fix the failure or error of one or more service components connected via the platform link application 208. The response may be a confirmation indicating whether a requested command or operation was successful.

The fourth logic 916 may be configured to provide (e.g., via the GUI 300), the response to the request (at 1016). For example, the IPC electronic processor 202 may be configured to provide the answer to a question in (or adjacent to) the chat box. In some instances, the IPC electronic processor 202 may provide one or more selectable prompts 310 in the control region 306. The one or more selectable prompts 310 may be, for example, possible operations or commands performed by the IPC electronic processor 202 that relate to the received request. The operations or commands may be, for example, operations or commands to fix the failure or error of the device 108 or cause the device 108 to perform some operation (for example, measure a sample). The one or more selectable prompts 310 may be provided as a list of recommended commands.

The fifth logic 920 may be configured to receive, via the GUI 300, an input indicating a request to execute the recommended command (at 1020). For example, user and input device and an input technique, a user or operator of the IPC 104 may select one of the one or more selectable prompts 310, thereby selecting a command for the IPC 104 to perform. In another instance, the one or more selectable prompts 310 merely includes a confirmation to execute a recommended command (e.g., "CONTINUE?" or "EXECUTE?").

The sixth logic 924 may be configured to identify what devices or communications are needed to execute the recommended command (at 1024). For example, when the GUI 300 receives the input to execute the recommended command, the IPC electronic processor 202 may apply one or more rules or mappings to identify how to make one or more API calls to accomplish the recommended command. For example, the IPC 104 (e.g., as part of the platform link application 208) may store rules that map particular commands to particular API calls for one or more devices 108 and/or instruments 112 associated with the IPC 104.

The seventh logic 928 may be configured to execute the recommended command, such as, for example, using the one or more APIs (at 1028). For example, the IPC electronic processor 202 interfaces with the device 108 using the identified APIs to perform the recommended command. In this manner, the command that was identified by the language model 226 is performed by the IPC 104.

In some implementations, when the response is a command, the fourth logic 916 (at 1016) and the fifth logic 920 (at 1020) may be skipped or omitted such that the IPC 104 (using the platform link application 208) automatically executes commands within requiring selection by a user within the GUI 300. Also, user authentication can be performed as part of responding to a query, such as, for example, as part of the first logic 904, second logic 908, third logic 912, fourth logic 916, fifth logic 920, sixth logic 924, seventh logic 928, or a combination thereof, may authenticate a user (including verifying an access level of the user) as part of providing a response to a user-submitted query or request. This authentication ensures that only authorized users are permitted to interact with the platform link application 208 and only users with appropriate authentication and access levels are permitted to obtain particular data or functionality as part of a response to a submitted query (e.g., run particular commands or operations). For example, in some embodiments, before providing recommended commands within the GUI 300 or in response to receiving a selection of a listed recommended command, the platform link application 208 may be configured to authenticate the user, such as, for example, by confirming an access level of the user submitting the request via an authentication service of the platform (e.g., as provided by the server 128). The authentication may be performed by the IPC 104, the platform link application 208, the server 128, or a combination thereof. In some implementations, the language model 226 may be configured to receive an authorization level of the user as an input alongside the request. The output provided by the language model 226 may then be a customized response based on the authorization level of the user. However, in other implementations, a response from the language model 226 may be verified against an access level of the user (e.g., via the platform link application 208) prior to being implemented or executed to ensure only authenticated users with appropriate access levels or rights are executing commands as recommended by the language model 226 as a response to a submitted query.

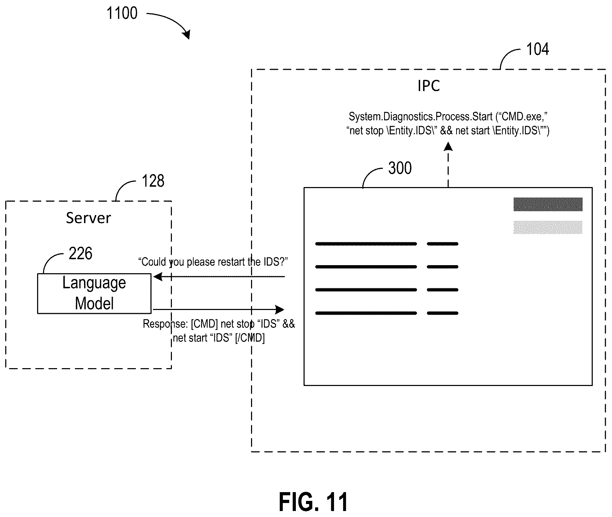

FIG. 11 illustrates additional details regarding implementation of the language model 226 and, in particular, illustrates an example workflow 1100 (e.g., a prompt-based workflow) for implementing the language model 226. As illustrated in FIG. 11, as one non-limiting example, the IPC 104 (i.e., the platform link application 208) receives a query of "Could you please restart the instrument diagnostic service?" through a GUI 300 provided via the platform link application 208. This query is transmitted to the language model 226, which, as noted above, may be installed on the server 128. The language model 226 may have been previously trained with a query and answer pair that included the query "Restart the IDS" and the corresponding answer that includes the following command instructions/command line (e.g., /CMD):
[CMD] net stop "Entity.IDS" && net start "Entity.IDS" [/CMD]

Thus, in response to receiving the query "Could you please restart the IDS" from the IPC 104, the language model 226 (which processes the received query with the tokenizer 228) generates an answer that includes the above instructions. This answer to transmitted to the IPC 104, which uses one or more rules or command mappings to identify how to execute the provided command. For example, for commands relating to diagnostics, the IPC 104 may be configured to make an API call to a system diagnostics application executed on the IPC 104 and may pass the received command instructions or a portion thereof (which may be processed or formatted accordingly to be compatible with the API call).

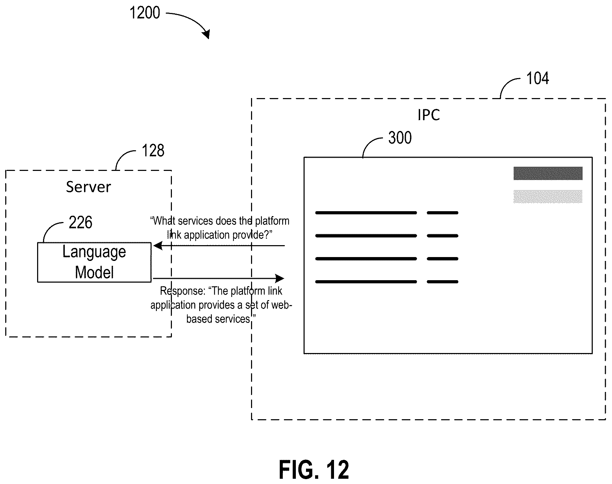

FIG. 12 illustrates further details regarding implementation of the language model 226 and, in particular, illustrates an example workflow 1200 for implementing the language model 226. As illustrated in FIG. 12, as one non-limiting example, the IPC 104 (i.e., the platform link application 208) receives a query of "What services does the platform link application provide?" through a GUI 300 provided via the platform link application 208. The language model 226 may have been previously trained with a query and answer pair that induced the corresponding answer "The platform link application provides a set of web-based services."

Thus, in response to receiving the query "What services does the platform link application provide?" from the IPC 104, the language model 226 (which processes the received query with the tokenizer 228) generates an answer providing information. This answer is transmitted to the IPC 104, which provides the answer to the query via the GUI 300.

The example method 500 of FIG. 5 primarily refers to training the language model 226 and storing the trained language model 226 in the server memory 220. However, the language model 226 may also be dynamically updated based on user interactions with the support system 100. For example, the language model 226 may be updated by the server electronic processor 222 based on the request associated with the platform link application 208 (at 1004) and/or the input indicating to execute the recommended command (at 1020). The language model 226 may be updated based on variations in requests, how common requests are, and how often recommended commands are executed.

In some examples, the IPC electronic processor 202 and/or the server electronic processor 222, in conjunction with the language model 226 and/or the platform link application 208, may validate user commands, such as those provided in workflow 1100 of FIG. 11. For example, the language model 226 receives a command from the user, such as "Have Device A perform Operation B." The language model 226 compares the command to the training documentation to determine whether Device A is capable of performing Operation B. When Device A is capable of performing Operation B, the language model 226 proceeds as described with respect to the workflow 1100. When Device A is not capable of performing Operation B, the language model 226 generates a response to the command indicating that the Device A is not capable of performing Operation B. The language model 226 may request that the user input a new query or command. In some instances, the language model 226 may identify one or more commands similar to Operation B which can be performed by the Device A that are provided as the one or more selectable prompts 310. In some instances, the IPC electronic processor 202 and/or the server electronic processor 222 are configured to validate user commands without interfacing with the language model 226. For example, the platform link application 208 may be configured to validate commands that are performable for a user.

In some instances, new devices 108 may be added to the support system 100. In such instances, the new device 108 may include an update for the language model 226, such as additional training documentation that is associated with the new device 108 to provide to the language model 226 over the network 120. In other instances, the device 108 has a software update for the language model 226 that is transmitted to the server 128 over the network 120. Additionally, devices 108 may be added to the support system 100 without being registered with the server 128. In such an instance, a user may query the language model 226 requesting that the new device 108 be registered with the server 128. The server memory 220 may identify documentation associated with the new device 108 or receive documentation directly from the new device 108 to update the language model 226 as part of the registration. The platform link application 208 then registers the new device 108 with the server 128, which allows services provided by the server 128 (i.e., the platform) to be used with the device 108.

Thus, examples provided herein provide assistance for understanding and using services associated with the platform link application as installed on an IPC, such as, for example, questions related to available services provided via the platform link application, starting and stopping services of the platform link application (e.g., based on authentication and authorization), identify if the platform link application has been onboarded with the server providing the platform, troubleshoot or identify issues with any of the provided services, or a combination thereof. Thus, the assistance user interfaces provided via the platform link application may provide a one-stop, user interactive AI driven tool to aid in responding to queries related to the platform link application and clarifies workflow queries of deployed services and helps with the performance of troubleshooting steps. For example, after the platform link application as installed on an IPC 104 is onboarded and devices 108 and/or instruments 112 are configured, a user may notice that changes (e.g., device or instrument changes) are not reflected via service provided via the platform (e.g., as accessed via the one or more user devices 130 through the server 128). Rather than manually going through extensive manuals for the platform client services, a query can be submitted through the assist tool of the platform link application 208 on the IPC 104 to run health or diagnostic check and find if there are any connection issues with the server 128. The language model 226 responds to such a query by providing command instructions for starting the requested diagnostics and may receive generated status reports and provide the status reports as a response to the query (E.g., through the GUI 300 receiving the initial query). These status reports can be used to devise and execute a troubleshooting plan, which may include restarting one or more services, which as described above with respect to the example of FIG. 11, may be performed through the assist tool of the platform link application 208. Accordingly, the AI drive assist tool of the platform link application 208 provides efficiencies in terms of time and computing resources while aiding in establishing managing platform links and maintaining proper authentication and authorization when performing automated actions in response to submitted queries. The tool also provides an enhanced customer experience, such as, for example, by allowing natural language question and answer interactions.

The following paragraphs provide various examples of the examples disclosed herein.

Clause 1. A scientific instrument support system, comprising: a server storing a trained language model; and an instrument computing device including: a display device configured to provide a user interface, a memory including a platform link application providing an interface between the instrument computing device and a platform of network-based services, a transceiver, and an electronic processor communicatively connected to the display device, the memory, and the transceiver, wherein the electronic processor is configured to: receive a request associated with the platform link application, transmit, with the transceiver, the request to the trained language model, receive, with the transceiver and from the trained language model, a recommended command associated with the request, confirm an access level of a user submitting the request, and in response to confirming the access level of the user: execute the recommended command.

Clause 2. The scientific instrument support system of clause 1, wherein the trained language model is trained using data pairs of questions and answers related to the platform link application.

Clause 3. The scientific instrument support system of any of clauses 1 to 2, wherein the electronic processor is configured to: provide, via a graphical interface, the recommended command, and receive an input confirming execution of the recommended command, wherein the electronic processor is configured to execute the recommended command in response to receiving the input and confirming the access level of the user.

Clause 4. The scientific instrument support system of clause 3, wherein the electronic processor is configured to provide, via the graphical interface, the recommended command by providing a list of a plurality of recommended commands.

Clause 5. The scientific instrument support system of clause 4, wherein the electronic processor is configured to receive the input confirming execution of the recommended command by receiving a selection of one of the plurality of recommended commands included in the list.

Clause 6. The scientific instrument support system of clause 5, wherein the electronic processor is further configured to update the trained language model based on the selection of the one of the plurality of recommended commands included in the list.

Clause 7. The scientific instrument support system of any of clauses 1 to 6, wherein the electronic processor is further configured to: receive a second request associated with the platform link application; transmit, with the transceiver, the second request to the trained language model, receive, with the transceiver and from the trained language model, a second recommended command associated with the second request, confirm the access level of a user submitting the second request, and in response to confirming the access level of the user: execute the second recommended command, wherein, to execute the second recommended command, the electronic processor is configured to: execute a diagnostic service of the platform link application, and provide a status report based on results of executing the diagnostic service.

Clause 8. The scientific instrument support system of any of clauses 1 to 7, wherein the recommended command is a restart command for restarting one or more of the network-based services.

Clause 9. The scientific instrument support system of any of clauses 1 to 8, wherein, to determine the access level associated with the user of the platform link application, the electronic processor is configured to provide identifying data of the user to an authentication service provided via the platform.

Clause 10. The scientific instrument support system of any of clauses 1 to 9, wherein the electronic processor is further configured to: receive a question associated with the platform link application, transmit, with the transceiver, the question to the trained language model, receive, with the transceiver and from the trained language model, a text-based answer to the question, and provide, via a graphical interface, the text-based answer.

Clause 11. A non-transitory computer readable medium storing instructions that, when executed by one or more electronic processing devices, perform a set of functions, the set of functions comprising: receiving a request associated with a platform link application, wherein the platform link application provides an interface between an instrument computing device and a platform of network-based services; transmitting, with a transceiver, the request to a trained language model; receiving, with a transceiver and from the trained language model, a recommended command associated with the request; confirming an access level of a user submitting the request; and in response to confirming the access level of the user, executing the recommended command.

Clause 12. The non-transitory computer readable medium of clause 11, wherein the set of functions further includes: providing, via a graphical interface, the recommended command; and receiving an input confirming execution of the recommended command, wherein executing the recommended command includes executing the recommended command in response to receiving the input and confirming the access level of the user.

Clause 13. The non-transitory computer readable medium of clause 12, wherein providing, via the graphical interface, the recommended command includes providing, via the graphical interface, a list of a plurality of recommended commands, and wherein receiving the input confirming execution of the recommended command includes receiving a selection of one of the plurality of recommended commands included in the list.

Clause 14. The non-transitory computer readable medium of clause 13, wherein the set of functions further includes: updating the trained language model based on the selection of the one of the plurality of recommended commands included in the list.

Clause 15. The non-transitory computer readable medium of any of clauses 11 to 14, wherein the set of functions further comprises: receiving a second request associated with the platform link application; transmitting, with the transceiver, the second request to the trained language model, receiving, with the transceiver and from the trained language model, a second recommended command associated with the second request, confirming the access level of a user submitting the second request, and in response to confirming the access level of the user: executing the second recommended command, wherein, to executing the second recommended command includes: executing a diagnostic service of the platform link application, and providing a status report based on results of executing the diagnostic service.

Clause 16. A scientific instrument support system, comprising: a memory including a platform link application providing an interface between an instrument computing device and a platform of network-based services, and an electronic processor communicatively connected to the memory, the electronic processor configured to: receive a request associated with the platform link application, provide the request to a trained language model, receive, from the trained language model, a recommended command associated with the request, confirm an access level of a user submitting the request, and in response to confirming the access level of the user: execute the recommended command.

Clause 17. The scientific instrument support system of clause 16, wherein the electronic processor is further configured to: identify one or more devices or communications associated with the recommended command, wherein, to execute the recommended command, the electronic processor is configured to execute the recommended command using the one or more devices or communications.

Clause 18. The scientific instrument support system of any of clauses 16 to 17, wherein the electronic processor is configured to provide, via a graphical interface, the recommended command, and receive an input confirming execution of the recommended command, wherein the electronic processor is configured to execute the recommended command in response to receiving the input and confirming the access level of the user.

Clause 19. The scientific instrument support system of clause 18, wherein the electronic processor is configured to: provide, via the graphical interface, the recommended command by providing a list of a plurality of recommended commands, and receive the input indicating a request to execute the recommended command by receiving a selection of one of the plurality of recommended commands included in the list.

Clause 20. The scientific instrument support system of any of clauses 16 to 19, wherein, to determine the access level associated with the user of the platform link application, the electronic processor is configured to: compare user identifier information associated with the user to a plurality of stored user profiles, and determine the access level associated with the user based on one of the plurality of stored user profiles associated with the user identifier information.

## Claims

1. A computer program containing instructions that, when executed by one or more electronic processing devices, perform a set of functions, the set of functions comprising:
receiving a request associated with a platform link application, wherein the platform link application provides an interface between an instrument computing device and a platform of network-based services;
transmitting, with a transceiver, the request to a trained language model;
receiving, with a transceiver and from the trained language model, a recommended command associated with the request;
confirming an access level of a user submitting the request; and
in response to confirming the access level of the user, executing the recommended command.

2. The computer program of claim 1, wherein the set of functions further includes:
providing, via a graphical interface, the recommended command; and
receiving an input confirming execution of the recommended command, wherein executing the recommended command includes executing the recommended command in response to receiving the input and confirming the access level of the user.

3. The computer program of claim 2, wherein providing, via the graphical interface, the recommended command includes providing, via the graphical interface, a list of a plurality of recommended commands.

4. The computer program of claim 3, wherein receiving the input confirming execution of the recommended command includes receiving a selection of one of the plurality of recommended commands included in the list.

5. The computer program of claim 3 or claim 4, wherein the set of functions further includes:
updating the trained language model based on the selection of the one of the plurality of recommended commands included in the list.

6. The computer program of any preceding claim, wherein the set of functions further comprises:
receiving a second request associated with the platform link application;
transmitting, with the transceiver, the second request to the trained language model,
receiving, with the transceiver and from the trained language model, a second recommended command associated with the second request,
confirming the access level of a user submitting the second request, and
in response to confirming the access level of the user:
executing the second recommended command,
wherein, to executing the second recommended command includes:
executing a diagnostic service of the platform link application, and
providing a status report based on results of executing the diagnostic service.

7. A non-transitory computer readable medium upon which is embodied the computer program of any of claims 1-6.

8. A scientific instrument support system, comprising:
a memory including a platform link application providing an interface between an instrument computing device and a platform of network-based services, and
an electronic processor communicatively connected to the memory, the electronic processor being configured to execute the instructions of the computer program of any one of claims 1-6

9. The scientific instrument support system of claim 8, wherein the electronic processor is further configured to:
identify one or more devices or communications associated with the recommended command,
wherein, to execute the recommended command, the electronic processor is configured to execute the recommended command using the one or more devices or communications.

10. The scientific instrument support system of claim 8 or claim 9, wherein, to determine the access level associated with the user of the platform link application, the electronic processor is configured to:
compare user identifier information associated with the user to a plurality of stored user profiles, and
determine the access level associated with the user based on one of the plurality of stored user profiles associated with the user identifier information.

11. A scientific instrument support system, comprising:
a server storing a trained language model; and
an instrument computing device including:
a display device configured to provide a user interface,
a memory including a platform link application providing an interface between the instrument computing device and a platform of network-based services,
a transceiver, and
an electronic processor communicatively connected to the display device, the memory, and the transceiver, wherein the electronic processor is configured to execute the instructions of the computer program of any one of claims 1-6.

12. The scientific instrument support system of claim 11, wherein the trained language model is trained using data pairs of questions and answers related to the platform link application.

13. The scientific instrument support system of claim 11 or claim 12, wherein the recommended command is a restart command for restarting one or more of the network-based services.

14. The scientific instrument support system of any one of claims 11-13, wherein, to determine the access level associated with the user of the platform link application, the electronic processor is configured to provide identifying data of the user to an authentication service provided via the platform.

15. The scientific instrument support system of any one of claims 11-14, wherein the electronic processor is further configured to:
receive a question associated with the platform link application,
transmit, with the transceiver, the question to the trained language model,
receive, with the transceiver and from the trained language model, a text-based answer to the question, and provide, via a graphical interface, the text-based answer.
